# EUROPEAN PATENT SPECIFICATION

(11) **EP 1 503 747 B1**
(45) Date of publication and mention of the grant of the patent: **26.07.2006**
(21) Application number: 03749856.5
(22) Date of filing: 16.04.2003
(51) Int. Cl.: A61K 31/196, A61K 9/00, A61K 31/195

(54) **DICLOFENAC-BASED COMPOSITION FOR THE TOPICAL TREATMENT OF OROPHARYNGEAL CAVITY DISORDERS**
ZUSAMMENSETZNG AUF DICLOFENAC-BASIS FÜR DIE TOPISCHE BEHANDLUNG VON ERKRANKUNGEN DES OROPHARYNX
COMPOSITION A BASE DE DICLOFENAC POUR TRAITEMENT LOCAL DE TROUBLES DE CAVITE OROPHARYNGEE

(30) Priority: 10.05.2002 IT MI20020986
(43) Date of publication of application: 09.02.2005
(73) Proprietor: AZIENDE CHIMICHE RIUNITE ANGELINI FRANCESCO A.C.R.A.F. S.p.A., 00181 Roma (IT)
(72) Inventor: PINZA, Mario, I-20094 Corsico (IT)
(74) Representative: Marchi, Massimo
(86) International application number: PCT/EP2003/004044
(87) International publication number: WO 2003/094905

(56) References cited:
- EP-A- 0 373 103
- EP-A- 0 521 393
- WO-A-92/00725
- US-A- 4 407 824
- US-A- 5 972 906

## Description

The present invention relates to a diclofenac-based composition for the topical treatment of oropharyngeal cavity disorders.

It is known that diclofenac [2-(2,6-dichloroanilino)phenylacetic acid] is a widely-used pharmaceutical product with anti-inflammatory, antipyretic and analgesic properties. It is mainly administered systemically in unmodified form or in the form of a salt thereof with mineral or organic bases.

However, its salts are virtually insoluble in water.

Example 2 of patent US-4 407 824 describes the preparation of the salt of diclofenac with tromethamine [tris(hydroxymethyl)methylamine], but does not specify its solubility in water and does not give an example of any pharmaceutical form containing the abovementioned salt.

The problem of the insolubility in water of diclofenac salts is also acknowledged in EP-A-0 521 393, which proposes to solve the said problem by means of the choline salt. This salt is described as a compound that is surprisingly soluble in water and suitable, inter alia, also for the preparation of mouthwashes.

However, the choline salt has the typical drawbacks of choline, which is well known for its unpleasant odour and taste.

These drawbacks are particularly unfavourable in the case of compositions for the topical treatment of oropharyngeal cavity disorders, for instance mouthwashes and oral sprays, which need to remain in contact with the mucosae for a relatively long period of time in order to exert their therapeutic effect.

Despite the addition of large amounts of ingredients capable of masking its taste [0.5% (w/w) of acesulfame and 35% (w/w) of sorbitol], compositions for the topical treatment of oropharyngeal cavity disorders based on the salt of diclofenac with choline are relatively unpalatable.

There is therefore still a great need for a diclofenac-based composition of pleasant or at the very least neutral taste, for the topical treatment of oropharyngeal cavity disorders.

Although A. Fini et al. have reported that the solubility in water of the tromethamine salt is considered to be 0.167 g in 100 ml (European J. Pharm. Sci. 4, 231, 1996), the tests conducted by the present inventor have demonstrated that amounts of diclofenac ranging from 0.071 to 0.142 g do not dissolve in 100 ml of water even in the presence of stoichiometric amounts (from 0.029 to 0.058 g, respectively) of tromethamine (Comparative Examples 1 and 2).

Surprisingly, it has now been found that the abovementioned compositions containing from 0.071 to 0.142 g of diclofenac with stoichiometric amounts (from 0.029 to 0.058 g, respectively) of tromethamine in 100 ml of water become clear and remain so for a long time if their pH is brought to 7-8 (Examples 1 and 2).

Also surprisingly, it has been found that the palatability of these solutions is good and that it is also very easy to improve it by means of modest amounts of standard flavouring agents and sweeteners.

One subject of the present invention is thus a composition for the topical treatment of oropharyngeal cavity disorders, characterized in that it comprises an aqueous solution of the salt of diclofenac with tromethamine, in which the amount of the said salt is of from 0.1% to 0.2% (w/w) and the pH is adjusted between 7 and 8.

The preferred concentration of the salt of diclofenac with tromethamine in the composition of the present invention is 0.1 % (w/w).

Advantageously, the abovementioned mouthwash comprises other standard ingredients, for instance ethanol, polyhydroxylated alcohols, complexing agents, preserving agents, humectants, sweeteners, flavouring agents, colouring agents and the like.

Typical examples of these ingredients are:
polyhydroxylated alcohols: glycerol, propylene glycol and polyethylene glycol;
complexing agents: sodium edetate;
preserving agents: methyl p-hydroxybenzoate and propyl p-hydroxybenzoate, sodium benzoate;
humectants: glyceryl polyethylene glycol ricinoleate;
sweeteners: sodium saccharinate, sorbitol, acesulfame and xylitol; gelling agents: block copolymers of polyethylene glycol and polypropylene glycol such as, for example, Poloxamer™ 407;
flavouring agents: mint flavouring agent, natural tutti frutti flavouring agent and grenadine flavouring agent;
colouring agents: quinoline yellow E 104 and patent blue E 131.

Typical examples of oropharyngeal cavity disorders which benefit from treatment with the composition of the present invention are: gingivitis, glossitis, stomatitis, aphthae, paradentosis, paradentitis, laryngitis, pharyngitis and mucositis caused by radiotherapy and chemotherapy. In addition, the composition of the invention is useful in the treatment of after-effects of dental and/or general surgery.

Preferred dosage forms of the composition of the present invention are mouthwashes and oral sprays.

These dosage forms can be readily prepared according to techniques known to pharmaceutical chemists, and include stages such as mixing, dissolution, sterilization and the like.

The following examples serve to illustrate the invention.

### Example 1

### Mouthwash A

100 g of Mouthwash A contains:

| | |
|---|---|
| salt of diclofenac with tromethamine* | 0.104 g |
| xylitol | 10.000 g |
| Poloxamer™ 407 | 0.500 g |
| sodium benzoate | 0.500 g |
| natural mint flavouring agent | 0.500 ml |
| aqueous solution of E 131 (1 mg/ml) | 0.200 ml |
| pH 7.8 phosphate buffer** qs | 100 g |
| pH | 7.6 |

| | |
|---|---|
| * equal to 0.074 g of acidic diclofenac | |
| ** one litre of solution in purified water contains: anhydrous dibasic sodium phosphate (5.803 g), anhydrous monobasic potassium phosphate (3.522 g) and 1 N sodium hydroxide (18.70 ml). | |

### Example 2

### Mouthwash B

100 g of Mouthwash B has the same composition as Mouthwash A except that:
- it also contains natural tutti frutti flavouring agent (0.04 ml) and natural grenadine flavouring agent (0.02 ml), and
- in place of 0.2 ml of aqueous solution of E 131 (1 mg/ml), it contains 0.25 ml of aqueous solution of E 124 (10 mg/ml).

### Comparative Example 1

### Mouthwash C

A mouthwash was prepared having the same composition as Mouthwash A, except that it contained purified water in place of the pH 7.8 phosphate buffer.

### Comparative Example 2

### Mouthwash D

A mouthwash was prepared having the same composition as Mouthwash B, except that it contained purified water in place of the pH 7.8 phosphate buffer.

### Stability

Mouthwashes A and B were found to be stable.

In contrast, Mouthwashes C and D released over time, especially under cold conditions, a precipitate of diclofenac.

This behaviour was entirely unexpected as regards the mouthwashes containing an amount of salt of diclofenac with tromethamine that is less than the solubility limit reported by Fini et al. (cited above).

## Claims

1. Composition for the topical treatment of oropharyngeal cavity disorders, **characterized in that** it comprises an aqueous solution of the salt of diclofenac with tromethamine, in which the amount of the said salt is of from 0.1 % to 0.2% (w/w) and the pH is adjusted between 7 and 8.

2. Composition according to Claim 1, **characterized in that** it contains 0.1% (w/w) of the salt of diclofenac with tromethamine.

3. Composition according to Claim 1 or 2, **characterized in that** it further comprises a sweetener selected from the group consisting of sodium saccharinate, sorbitol, acesulfame and xylitol.

4. Composition according to any one of the preceding Claims 1 to 3, **characterized in that** it further comprises a preserving agent selected from the group consisting of sodium benzoate, methyl p-hydroxybenzoate and propyl p-hydroxybenzoate.

5. Composition according to any one of the preceding Claims 1 to 4, **characterized in that** it further comprises a gelling agent consisting of a block copolymer of polyethylene glycol and polypropylene glycol.

6. Composition according to any one of the preceding Claims 1 to 5, **characterized in that** it further comprises a pharmaceutically acceptable flavouring agent.

7. Composition according to any one of the preceding Claims 1 to 6, **characterized in that** it further comprises a pharmaceutically acceptable colouring agent.

8. Composition according to any one of the preceding Claims 1 to 7, **characterized in that** it is used in the treatment of gingivitis, glossitis, stomatitis, aphthae, paradentosis, paradentitis, laryngitis, pharyngitis, mucositis of the oral cavity caused by radiotherapy and chemotherapy, and of after-effects of dental and/or general surgery.

## Patentansprüche

1. Zubereitung zur topischen Behandlung von Erkrankungen des Mundrachenraums, **dadurch gekennzeichnet, dass** sie eine wässrige Lösung des Salzes von Diclofenac mit Tromethamin umfasst, wobei die Menge des Salzes 0,1 Gew.-% bis 0,2 Gew.-% und der pH auf 7 bis 8 eingestellt ist.

2. Zubereitung nach Anspruch 1, **dadurch gekennzeichnet, dass** sie 0,1 % Gew.-% des Salzes von Diclofenac mit Tromethamin enthält.

3. Zubereitung nach Anspruch 1 oder 2, **dadurch gekennzeichnet dass** sie außerdem ein Süßungsmittel umfasst, das unter Natriumsaccharinat, Sorbitol, Acesulpham und Xylitol ausgewählt ist.

4. Zubereitung nach einem der vorhergehenden Ansprüche 1 bis 3, **dadurch gekennzeichnet, dass** sie außerdem ein Konservierungsmittel umfasst, das unter Natriumbenzoat, Methyl-p-hydroxybenzoat und Propyl-p-hydroxybenzoat ausgewählt ist.

5. Zubereitung nach einem der vorhergehenden Ansprüche 1 bis 4, **dadurch gekennzeichnet, dass** sie außerdem ein Geliermittel umfasst, das aus einem Blockcopolymer von Polyethylenglykol und Polypropylenglykol besteht.

6. Zubereitung nach einem der vorhergehenden Ansprüche 1 bis 5, **dadurch gekennzeichnet, dass** sie außerdem einen pharmazeutisch verträglichen Aromastoff umfasst.

7. Zubereitung nach einem der vorhergehenden Ansprüche 1 bis 6, **dadurch gekennzeichnet, dass** sie außerdem ein pharmazeutisch verträgliches Farbmittel umfasst.

8. Zubereitung nach einem der vorhergehenden Ansprüche 1 bis 7, **dadurch gekennzeichnet, dass** man sie zur Behandlung von Gingivitis, Glossitis, Stomatitis, Aphthen, Parodontose, Parodontitis, Laryngitis, Pharyngitis, Mucositis der Mundhöhle nach Radiotherapie und Chemotherapie, und von Nachwirkungen einer Dental- und/oder allgemeinen Chirurgie verwendet.

## Revendications

1. Composition pour le traitement topique des troubles de la cavité buccopharyngée, **caractérisée en ce qu'**elle comprend une solution aqueuse du sel du diclofénac avec la trométhamine, dans laquelle la quantité dudit sel est de 0,1 % à 0,2 % (masse/masse) et le pH est ajusté entre 7 et 8.

2. Composition selon la revendication 1, **caractérisée en ce qu'**elle contient 0,1 % (masse/masse) du sel du diclofénac avec la trométhamine.

3. Composition selon la revendication 1 ou 2, **caractérisée en ce qu'**elle contient en outre un édulcorant choisi dans le groupe constitué par le saccharinate de sodium, le sorbitol, l'acésulfame et le xylitol.

4. Composition selon l'une quelconque des revendications 1 à 3 qui précèdent, **caractérisée en ce qu'**elle contient en outre un agent conservateur choisi dans le groupe constitué par le benzoate de sodium, le p-hydroxybenzoate de méthyle et le p-hydroxybenzoate de propyle.

5. Composition selon l'une quelconque des revendications 1 à 4 qui précèdent, **caractérisée en ce qu'**elle contient en outre un agent gélifiant constitué par un copolymère bloc du polyéthylèneglycol et du polypropylèneglycol.

6. Composition selon l'une quelconque des revendications 1 à 5 qui précèdent, **caractérisée en ce qu'**elle contient en outre un agent aromatisant pharmaceutiquement acceptable.

7. Composition selon l'une quelconque des revendications 1 à 6 qui précèdent, **caractérisée en ce qu'**elle contient en outre un agent colorant pharmaceutiquement acceptable.

8. Composition selon l'une quelconque des revendications 1 à 7 qui précèdent, **caractérisée en ce qu'**elle est utilisée dans le traitement d'une gingivite, d'une glossite, d'une stomatite, d'aphtes, d'une parodentose, d'une parodentite, d'une laryngite, d'une pharyngite, d'une mucosite de la cavité buccale provoquées par une radiothérapie et une chimiothérapie, et constituant une séquelle d'un traitement de chirurgie dentaire et/ou de chirurgie générale.
